# EUROPEAN PATENT APPLICATION

(11) **EP 0 645 447 A2**
(43) Date of publication of application: **29.03.1995**
(21) Application number: 94114103.8
(22) Date of filing: 08.09.1994
(51) Int. Cl.: C12M 1/30

(54) **Specimen collecting and transport system**

(30) Priority: 23.09.1993 US 125703
(71) Applicant: Becton Dickinson and Company, Franklin Lakes, New Jersey 07417-1880 (US)
(72) Inventor: Monthony, James F., Baltimore, MD 21209 (US); Livingston, Dwight, Baltimore, MD 21204 (US)
(74) Representative: Selting, Günther, Dipl.-Ing.

(57) **Abstract**

A sleeve for use with a device for collecting and transporting biological specimens. The sleeve is used to exert pressure to the collection device so as to break the ampoule. The sleeve comprises means for limiting over compression of the collection device. The sleeve provides an environment to maintain the substantial viability of a specimen during transport in a collection device.

## Description

### BACKGROUND OF THE INVENTION

### 1. Field of the Invention

This invention relates to a device and method for breaking ampoules. In particular the device may be used for breaking ampoules in an apparatus for collecting and transporting biological specimens.

### 2. Description of Related Art

An element common to most devices for collecting and transporting biological specimens is an ampoule in a container that can be broken to release medium into the container to keep the swab and sample moist. A typical collecting and transporting device has been described in several publications and most specifically in U.S. Patent No. 4,014,748.

A commercially available device for collecting and transporting biological specimens is the CULTURETTE® Collection and Transport System (trademark of Becton, Dickinson and Company) sold by Becton Dickinson Microbiology Systems, Cockeysville, Maryland. The CULTURETTE device is for collecting and transporting a biological sample with a protective sleeve or skirt surrounding the container where the ampoule, which is to be broken for use, is contained.

A problem that has confronted users of collection and transport devices is maintaining the viability and preventing the contamination of any biological sample which is collected. In spite of the use of a high level of skill and care in collecting the specimen so as to prevent contamination of the specimen, viability of the microorganisms is not always assured by the use of the prior art collection devices.

With the increased emphasis on the efficacy of medical products, a need exists for an improved device and method for effectively and efficiently handling the frangible ampoules in collection devices. The improved device would better protect the specimen during handling and use and would be comparatively simple and inexpensive to manufacture as compared to available devices.

### SUMMARY OF THE INVENTION

The present invention is a sleeve for use with a device for collecting and transporting biological specimens. The sleeve substantially prevents the specimen collected in the device from being exposed to the outside environment.

The sleeve preferably comprises two opposing members spaced sufficiently from one another. The opposing members extend from a forward end to a rearward end. Desirably the opposing members are spaced sufficiently from one another by collapsible sidewalls that extend from the forward end to the rearward end.

Preferably each member comprises a wall with an outer surface, an inner surface and an overhanging edge. The collapsible sidewalls extend between the inner surface of each member. Most preferably, the collapsible sidewalls are connected to each member by a living hinge.

The collapsible sidewalls comprise an inner surface and an outer surface. The inner surface of the members and the inner surface of the collapsible sidewalls face each other to form a cavity.

Desirably, the collapsible sidewalls have a curved inner wall surface in the shape of an arch, oval or the like. Preferably, the inner walls of the sidewalls are the same or symmetrical in shape. Most preferably, the collapsible sidewalls comprise an upper section and a lower section that is separated by a living hinge and means for limiting the compression of the members.

The sleeve may be permanently affixed to a collection device or, in an alternate embodiment, be removably disposed on the collection device and have the capability to be reused on another collection device.

The sleeve is preferably used with a collection device that comprises a tubular housing, a specimen collector and a frangible ampoule. The frangible ampoule holds medium that may be released into the tubular housing.

Most preferably, the sleeve encompasses the bottom of the collection apparatus wherein the frangible ampoule of the collection device is housed. The members of the device are compressed to provide appropriate pressure to the collection device to break the ampoule.

The collection device may be placed or attached in the cavity between the inner surfaces while a gap is maintained between the collection device and the inner surfaces of the members and the collapsible sidewalls.

Preferably, the inner wall surfaces of the members comprise a flat surface and each flat surface preferably has a protrusion. The protrusions assist in aligning the sleeve on the bottom section of the collection device where the ampoule is located.

To collect and transport a specimen, the specimen collector is removed from the tubular housing. A site, such as the area of the throat, nose, ears, mouth, wound or operative sites, is sampled by contacting the site with the swab of the specimen collector. The specimen is removed from the site with the swab. Thereafter the specimen collector is preferably returned to the tubular housing with the swab being preferably positioned in contact with a swatch or pledge. The sleeve is then positioned on the bottom section of the collection device where the ampoule is located. The user then effects a force, such as finger pressure, on the two opposing members of the sleeve. This in turn compresses the sleeve so that the sidewalls of the sleeve collapse, the cavity of the sleeve is reduced so there is not sufficient area for the collection device and as a result the ampoule is ruptured. The ruptured ampoule releases medium into the housing.

The sleeve is disposable, or may be reusable and is preferably made of an optically clear plastic but may also be made of an opaque plastic.

The sleeve of the present invention improves the standard collection type device in that microorganisms that are obtained by the collection device may remain substantially viable and uncontaminated through the entire specimen collection, transport, storage and identification phases.

### DESCRIPTION OF THE DRAWINGS

FIG. 1 is a perspective view of the sleeve.

FIG. 2 is a cross sectional view of FIG. 1 taken along line 2-2 thereof.

FIG. 3 is a perspective view illustrating a device for collecting and transporting specimens.

FIG. 4 is a perspective view illustrating the device for collecting and transporting specimens of FIG. 3 with the sleeve of FIG. 1.

FIG. 5 is a cross-sectional view of FIG. 4 taken along lines 5-5.

FIG. 6 is the cross sectional view of FIG. 5 illustrating the breaking of the ampoule.

FIG. 7 is a perspective view illustrating a system for collecting and transporting specimens comprising at least two ampoules.

### DETAILED DESCRIPTION

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

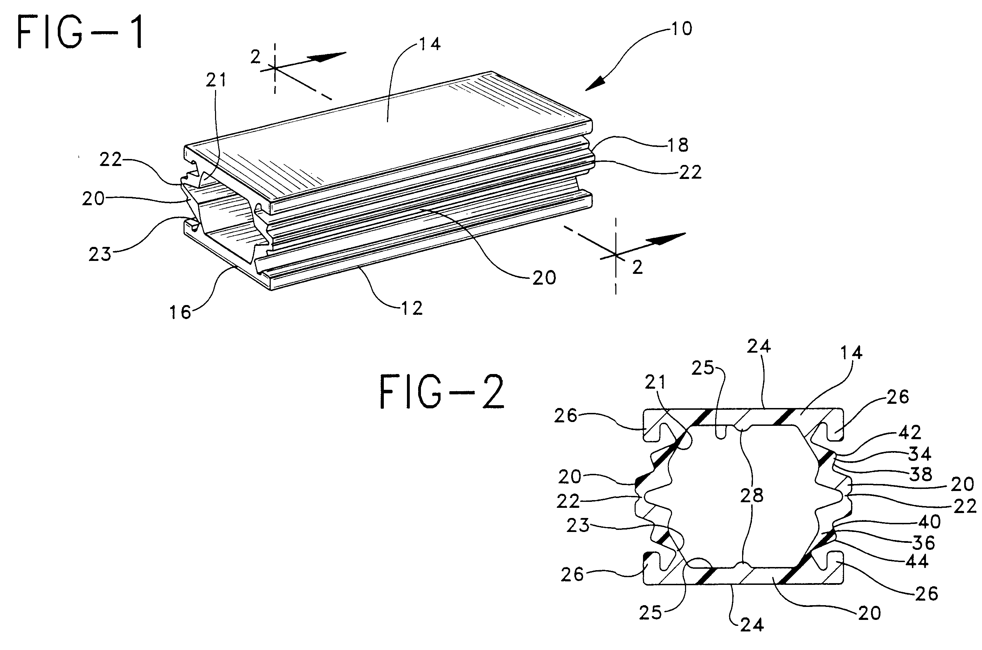

The preferred sleeve **10** of the present invention is illustrated in FIG. 1 wherein the sleeve has two movable and opposing members **12** and **14** that extend from a forward end **16** to a rearward end **18** and are connected by collapsible sidewalls **20**.

As shown in FIG. 2, each member has an outer surface **24**, an inner surface **25**, an overhanging edge **26** and a protrusion **28** on the inner surface. Collapsible sidewalls **20** have three living hinges **21**, **22** and **23**. Living hinge **21** separates collapsible sidewall **20** from member **14** and living hinge **23** separates collapsible sidewall **20** from member **12**. Collapsible sidewalls **20** each have an upper section **34** and a lower section **36** that are separated by living hinge **22**. The outer surface **38** of upper section **34** and the outer surface **40** of lower section **36** have a stop element **42** and **44** respectively. The inner surfaces of the members and the collapsible sidewalls face each other to form a cavity **46**.

The sleeve may be made of plastic or other flexible material. The sleeve is desirably translucent and most preferably transparent.

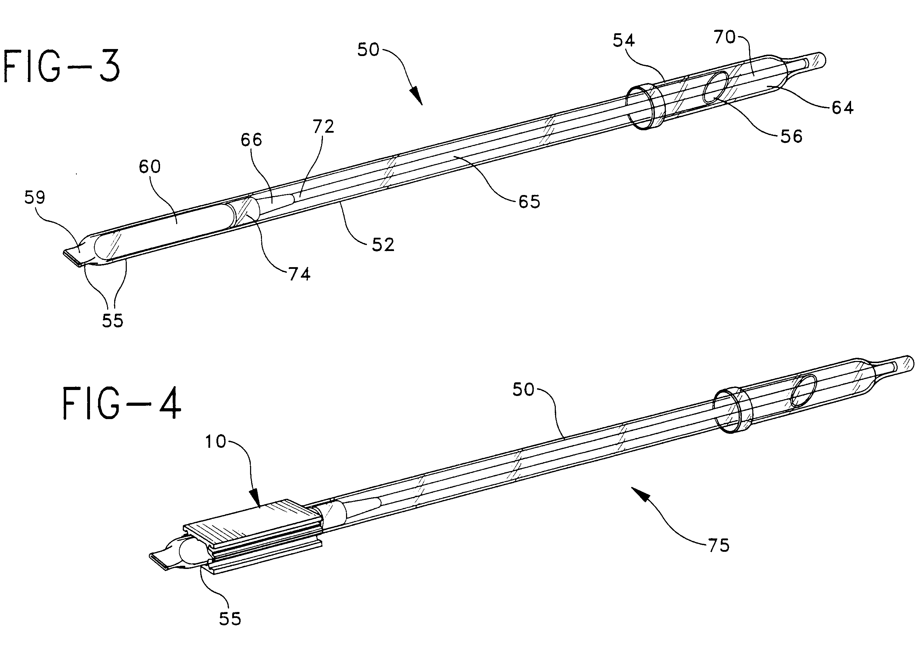

The sleeve is used with a typical collection device **50** as illustrated in FIG. 3. Device **50** preferably comprises a tubular housing **52** and a specimen collector **54**. Tubular housing **52** has a closed end extremity **55** and an opposite open end extremity **56**. The tubular housing is sealed at closed end extremity **55**, leaving surface **59** to which may be affixed by heat stamp, imprinting or other means a lot number or identification to enable traceability throughout the life of device **50**.

Device **50** also includes a cap **64**, a shaft **65**, and a swab **66**. Cap **64** is preferably removably attachable to the open end extremity of the tubular housing. The cap is preferably adapted to telescope snugly, but slidably, over the open end extremity of the tubular housing before and after swab **66** is used.

Shaft **65** comprises a first end **70** and a second end **72**. First end **70** is preferably connected to the cap and second end **72** is preferably connected to swab **66**. Swab **66** is preferably made from soft and absorbent materials including but not limited to suitable fibrous material such as cotton, polyester fibers or the like.

Disposed within the tubular housing and adjacent the closed end extremity is frangible ampoule **60**. The frangible ampoule preferably holds liquid medium which may be released inside the tubular housing. The liquid in the ampoule may be a transport medium which provides an environment in which the specimen can remain substantially viable. Frangible ampoule **60** is preferably made of, but not limited to, glass or some other frangible or rupturable material which is substantially non-reactive with the liquid therein. The frangible ampoule may be ruptured or broken upon application of pressure to the outer surface of the tubular housing section containing the frangible ampoule. In this way, the frangible ampoule may be opened and the liquid therein freed.

The tubular housing may be an optically clear plastic at the open end extremity and an opaque plastic in the area enclosing the frangible ampoule. The colored plastic highlights where the frangible ampoule is located or the type of medium enclosed in the ampoule.

Preferably, the tubular housing is made of easily compressible material so that the frangible ampoule, if employed in the device, may be crushed simply by applying pressure to the tubular housing.

In order to facilitate assembly of the device, frangible ampoule **60** fits rather loosely within the tubular housing adjacent the closed extremity. To retain the loose frangible ampoule in this position and also to restrain flow of released liquid, an absorbent plug **74** is located within the tubular housing. The absorbent plug may be tightly telescoped within the tubular housing and preferably abuts the frangible ampoule to prevent the latter from sliding.

Absorbent plug **74** may be made of cotton-like material or any other suitable material having absorbing properties, for properly restraining flow and/or fragments or particulate matter.

The swab is also preferably in contact with the absorbent plug and thus, as liquid is released from the frangible ampoule, the absorbent plug is moistened and in turn conducts the moisture to the swab. The liquid provides a moist environment for the specimen on the swab, to keep the specimen substantially viable during transport and to prevent dehydration of the specimen. In addition, the absorbent plug may substantially prevent fragments of the frangible ampoule from collecting on the swab after the frangible ampoule has been broken.

In some cases, the absorbent plug may not be employed in the tubular housing, however, it is a preferred element, especially when the liquid in the ampoule has a low viscosity. When the liquid has a low viscosity, the metering effect of the absorbent plug prevents the liquid and portions of the specimen from spreading onto the shaft or onto the interior face of the cap and thus prevents contamination of these parts.

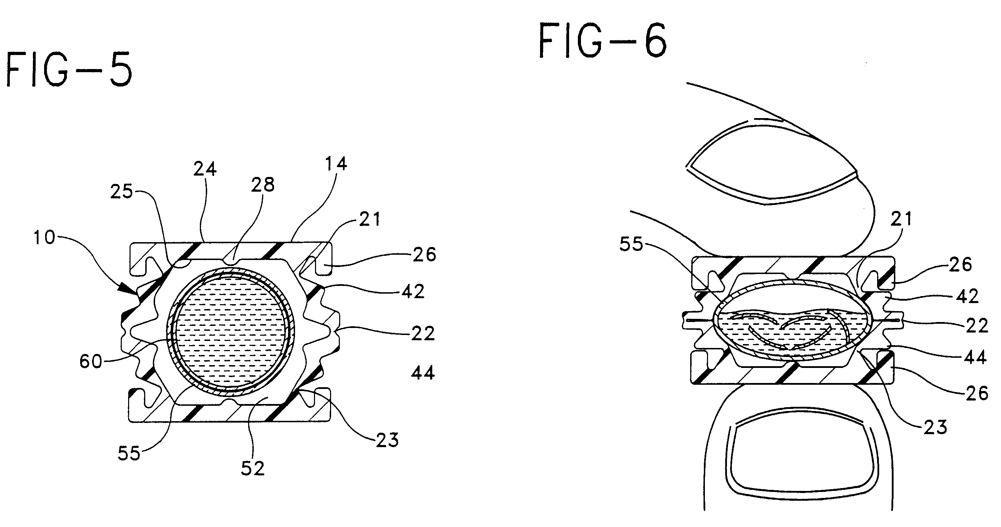

As shown in FIG. 4, sleeve **10** is removably attached to collection device **50** at and around the closed end extremity of the device where the frangible ampoule is housed so as to form a collecting and transporting system **75**. As shown in FIG. 5, the closed end extremity of the device is located within cavity **52** of the sleeve. The sleeve substantially surrounds the ampoule while having a space between the edges of the members. The housing may if desired, be secured to the inner surfaces of the sleeve with an adhesive.

To collect a specimen, cap **64** is removed from tubular housing **52** and swab **66** is pulled out of the tubular housing. A particular body passage of the patient then is swabbed with swab **66** to obtain a specimen. Thereafter, the swab with the specimen is returned to the tubular member with the swab being positioned in contact with absorbent plug **74**. The user then effects a force on the tubular housing at the locations marked with indicia on the guard arrangement to bring the edges of the members together. In turn the cavity is compressed or reduced so there is not sufficient area for the ampoule. The compressed or reduced cavity in turn effects a force on the ampoule so that the ampoule breaks and liquid is released. The liquid moistens the absorbent plug which, in turn, moistens the swab to keep the specimen in suitable condition until it reaches, for example, a laboratory or related facility for testing.

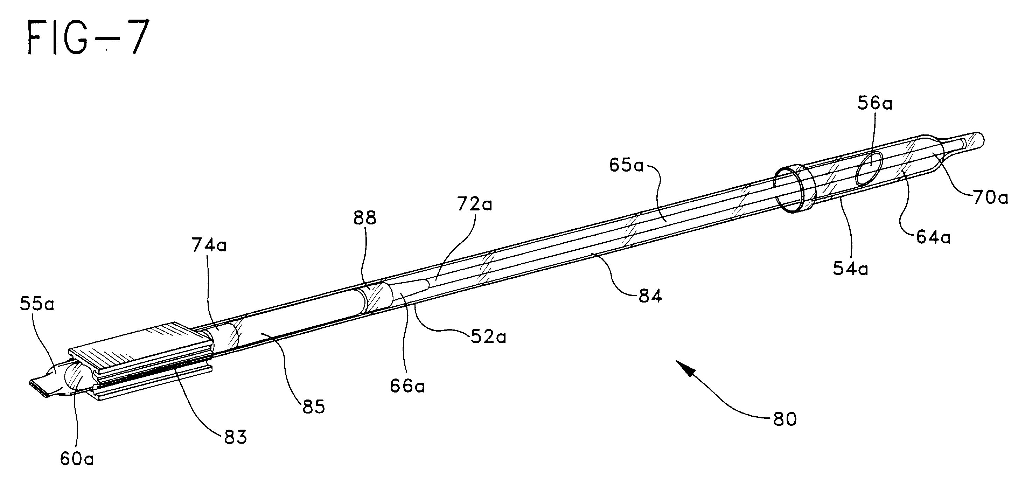

As shown in FIG. 6, the user effects a force, such as finger pressure, on the outer surface of each member. In turn, the upper section and lower section of the collapsible sidewalls meet, each stop element meets with the respective overhanging edge of each member, and the cavity of the sleeve is compressed or reduced. This causes the tubular housing to be compressed and the frangible ampoule to be ruptured. Stop elements **42** and **44** and overhang edge **26** prevent further movement of the sleeve and prevent over compression of the tubular housing thereby substantially eliminating contamination of the specimen.

Since the sleeve is most preferably made of a flexible material, after the user removes their fingers from the sleeve, the sleeve will revert back to its original shape and orientation so that it can be reused.

An alternate embodiment of a collection device is shown in FIG. 7, includes many components of FIGS. 1-6. Accordingly, similar components performing similar functions will be numbered identically to those components of FIGS. 1-6, except that a suffix "a" is used to identify those similar components in FIG. 7.

FIG. 7 illustrates an alternate embodiment of the invention, collecting and transporting system **80**, comprising a sleeve **82** and a collection device **84**. Collection device **84** has disposed within tubular housing **52a**, adjacent absorbent plug **74a**, a second frangible ampoule **85**. Frangible ampoule **85** is preferably made, but not limited to the same materials as frangible ampoule **60a**. Second frangible ampoule **85** preferably holds a test reagent which may be released inside the tubular housing. The test reagent may be used to detect and identify microorganisms and for receiving detectable antigen in the device. To retain the second frangible ampoule in position and also to restrain flow of released test reagent, a second absorbent plug **88** is located within the tubular housing. The absorbent plug is tightly telescoped within the tubular housing and abuts the second frangible ampoule to prevent the latter from sliding.

Sleeve **82** is removably attached to the collection device at and around the closed end extremity of the device where frangible ampoule **60a** is housed. The user ruptures frangible ampoule **60a** with sleeve **82** as described above and as shown in FIG. 6. The sleeve is then returned to its original orientation and is then removably attached to the collection device where the second frangible ampoule is housed. The user then ruptures the second frangible ampoule in the same manner as the first frangible ampoule was ruptured above.

The test reagent that is preferably held in the second frangible ampoule may be one or more of various well known test reagents. The particular test reagent used may be chosen on the basis of the particular type of species being identified. A test reagent such as N,N,N,N-tetra-methyl-p-phenylenediamine dihydrochloride may be used as disclosed in U.S. Patent 3,876,503, which is hereby incorporated by reference, for detecting gonorrhea. Other test reagents such as di-methyl-amino-cinnaminaldehyde, beta-d-galactosidase substrates, gamma-glutamylamino peptidase and prolylamine peptidase may also be used as disclosed in U.S. Patent No. 4,767,702 which is hereby incorporated by reference for detecting specific species of the genus Neisseria. Further test reagents, may include, but are not limited to, hippuric acid for detecting Group B Streptococcus, L-pyrrolidonyl-beta-naphthylamide and esculin for detecting Group A Streptococcus, acid or mineral acid and sodium nitrite for extracting Group A Streptococcus antigen and tris-buffer, sodium chloride, EDTA, sodium azide and N-acetyl-cysteine for extracting Respiratory Syncytial Virus (RSV) antigen.

It will be understood by practitioners-in-the-art that multiple ampoules may be disposed within the tubular housing of the collection device wherein each ampoule is separated by an absorbent plug. The number of frangible ampoules used is dependent on the need associated with the specimen collected and/or the particular test or immunoassay to be performed.

The collection devices described herein may be used for providing viable specimens for in vitro diagnostic testing methods, immunoassays for detecting and identifying microorganisms and for extracting detectable antigen. The collection devices may also be used in clinical situations to extract bacteria or other microorganisms from a clinical specimen for inoculation onto or into primary isolation media. The bacteria or microorganisms extracted by the collection devices may also be used for immunological or DNA/RNA probe testing or other tests to determine the identity and/or antimicrobial susceptibility pattern of the etiological agent.

The sleeve may be made of varying lengths so as to accommodate collection devices that may comprise more than one ampoule to be broken.

The following examples are not limited to any specific embodiment of the invention, but are only exemplary.

### EXAMPLE 1

### EXTRACTION OF GROUP A STREPTOCOCCUS ANTIGEN FROM A THROAT SPECIMEN

A throat site is contacted with a swab and a specimen, possibly containing Group A Streptococcus, is removed. The swab is inserted into a collection device with a tubular housing and two ampoules disposed within. A sleeve is thereafter removably disposed around the housing wherein the ampoules are disposed.

The first ampoule contains an acid or mineral acid such as acetic acid, citric acid or hydrochloric acid and the second ampoule contains sodium nitrite.

The first and second ampoules are broken in succession by the sleeve to permit the formation of nitrous acid to cause the extraction of Group A Streptococcus antigen if present for further testing by an immunological based assay such as DIRECTIGEN® Rapid Group A Strep Test (trademark of Becton, Dickinson and Company, Franklin Lakes, NJ) sold by Becton Dickinson Microbiology Systems, Cockeysville, MD.

### EXAMPLE 2

### RAPID PRESUMPTIVE CHROMOGENIC/COLORIMETRIC TEST FOR NEISSERIA GONORRHOEAE IN MALE URETHRAL DISCHARGE

A swab is inserted into a collection device with a tubular housing having two ampoules disposed within. A sleeve is removably disposed around the housing wherein the ampoules are disposed.

The first ampoule contains a saline wetting agent and the second ampoule contains the reagent N,N,N,N-tetra-methyl-p-phenylenediamine dihydrochloride.

The first ampoule is broken by the sleeve to release the wetting agent onto the swab. The swab is then removed from the device to collect a male urethral discharge specimen. The swab is reinserted into the collection device with the collected specimen.

The second ampoule is then broken by the sleeve to release the reagent. If N. gonorrhoeae is present in the specimen, the reagent will turn purple or blue/purple.

## Claims

1. A sleeve for use with a device for collecting and transporting specimens comprising of:
two movable and opposing members wherein each member comprises a forward end, a rearward end, an inner surface and an outer surface;
collapsible sidewalls that extend between said members and comprise an inner surface and an outer surface; and
a cavity between said opposing inner surfaces of said members and said sidewall, said cavity being of sufficient size to encompass at least one ampoule;
wherein said collapsible sidewalls each comprise at least one living hinge centrally located in said collapsible sidewalls;
wherein said collapsible sidewalls further comprise an upper section and a lower section, separated by said living hinge; and
wherein said upper section and said lower section comprise means for limiting the compression of said members, thereby preventing over compression of the device for collecting and transporting biological specimens and substantially eliminating contamination of the specimen.

2. The sleeve of Claim 1 wherein said members further comprise a protrusion on said inner surface.

3. The sleeve of Claim 2 wherein said means for limiting the compression of said members comprises overhanging edges on each of said members extending from said forward end to said rearward end toward the opposing member.

4. The sleeve of Claim 3 wherein said means for limiting the compression of said members further comprises stop means located in said collapsible sidewalls between said living hinge and said member.

5. An apparatus for collecting and transporting biological specimens comprising of:
an elongated tube having an open end and an opposite closed end;
a specimen collector disposed within said tube comprising a cap, a shaft connected to said cap and a swab connected to said shaft;
at least one ampoule disposed within said tube and located adjacent to said closed end of said tube; and
a sleeve removably disposed on said closed end of said housing comprising two movable and opposing members wherein each member comprises a forward end, a rearward end, an inner surface and an outer surface, collapsible sidewalls extending between said members and wherein said collapsible walls comprise an inner surface and an outer surface, and a cavity between said opposing inner surfaces of said members, said cavity being of sufficient size to encompass said at least one ampoule; and said collapsible sidewalls; wherein said collapsible sidewalls each comprise at least one living hinge centrally located in said collapsible sidewalls; said collapsible sidewalls further comprise an upper section and a lower section, separated by said living hinge; and wherein said upper section and said lower section comprise means for limiting the compression of said members.

6. The apparatus of Claim 5 wherein said members further comprise a protrusion on said inner surface.

7. The apparatus of Claim 6 wherein said means for limiting the compression of said members comprises overhanging edges on each of said members extending from said forward end to said rearward end toward the opposing member.

8. The apparatus of Claim 7 wherein said means for limiting the compression of said members further comprises stop means located in said collapsible sidewalls between said living hinge and said member.

9. A method for collecting and transporting biological samples comprising:
(a) providing an apparatus for collecting and transporting biological specimens having an elongated tubular housing extending from an open end to an opposite closed end, a specimen collector disposed within said housing comprising a cap, a shaft connected to said cap and a swab connected to said shaft, at least one ampoule disposed within said tube and located adjacent to said closed end of said tube; and; a sleeve removably disposed on said closed end of said housing, comprising two movable and opposing members wherein each member comprises a forward end, a rearward end, collapsible sidewalls extending between said members and wherein said collapsible sidewall comprises an inner surface and an outer surface, a cavity between said inner surfaces of said members, said cavity being of sufficient size to encompass said at least one ampoule; and wherein said collapsible sidewalls each comprise at least one living hinge centrally located in said collapsible sidewalls; said collapsible sidewalls further comprise an upper section and a lower section, separated by said living hinge; and wherein said upper section and said lower section comprise means for limiting the compression of said members, an ampoule containing a culture-sustaining medium disposed within the closed end of said housing and within said cavity of said sleeve;
(b) contacting a sampling site with said swab;
(c) obtaining a specimen from said site with said swab;
(d) inserting the swab with the specimen into said housing;
(e) closing said housing with said cap to protect said swab and said specimen from the environment external of said housing;
(f) applying force to said outer surfaces of said members, reducing said cavity, rupturing said ampoule and engaging said means for limiting the compression of said members;
(g) releasing medium into said housing; and
(h) subjecting said specimen to said medium without overcompressing the tube, thereby substantially eliminating contamination of the specimen.
